# EUROPEAN PATENT SPECIFICATION

(11) **EP 1 350 467 B1**
(45) Date of publication and mention of the grant of the patent: **03.01.2007**
(21) Application number: 02258197.9
(22) Date of filing: 28.11.2002
(51) Int. Cl.: A61B 6/02, A61B 6/00

(54) **Motion image processing apparatus and method**
Vorrichtung und Verfahren zum Verarbeiten von bewegten Bildern
Dispositif et méthode de traitement d'images en mouvement

(30) Priority: 03.04.2002 JP 2002101374; 03.04.2002 JP 2002101209; 26.08.2002 JP 2002245284
(43) Date of publication of application: 08.10.2003
(73) Proprietor: CANON KABUSHIKI KAISHA, Tokyo (JP)
(72) Inventor: Tsujii, Osamu, Canon Kabushiki Kaisha, Ohta-ku, Tokyo (JP)
(74) Representative: Beresford, Keith Denis Lewis

## Description

The present invention relates to a method and apparatus, apparatus for processing image data and displaying a motion image, as also a program product and a computer-readable storage medium, storing program instructions thereof.

Systems that have a semiconductor image sensor with a large area for radiation imaging an object (a subject) have been developed. In comparison to a conventional radiation photographing system, such a system has a practical advantage of recording an image in a wide range of radiation exposure. Specifically, X rays having a very wide dynamic range are captured as an electrical signal using a photoelectric converter, and the electrical signal is then converted into a digital signal. By processing the digital signal, a radiation image as a visible image is output to a recording medium, such as a photosensitive material, or to a display such as a CRT (Cathode Ray Tube). Even if the level of radiation changes slightly, an excellent radiation image can be obtained.

In image capturing using a semiconductor sensor, a breathing moving-state lung imaging in which the breathing lungs are imaged is expected to provide new medical information instead of image diagnosis based on a conventional still image. Breathing moving-state lung imaging refers to imaging in which images are captured in the form of a motion image from a state in which the lungs expand in inspiration to a state in which the lungs contract in expiration. Preferably, the lungs are imaged throughout one respiration period from the expansion time to the contraction time of the lungs.

Unlike imaging where the breathing held, imaging with the lung respiring presents difficulty in collecting data. The difficulty is in matching the respiration cycle precisely with the continuous capturing of the motion image (for example, with imaging starting with inspiration and ending with expiration). Even if a subject is instructed to start inspiring at the beginning of imaging, a delay depending on the subject takes place. In particular, aged people or feeble patients, typically with low physical strength, suffer from a significant delay. If the respiration phase is different at the start time of motion image from patient to patient, a physician is unable to determine a diagnosis method. Therefore, it takes extra time for the physician to diagnose the patient from resulting images.

To eliminate variations at the beginning, a sensor for detecting respiration may be used. The sensor can be used to time control the start and end of imaging. This requires the sensor to be mounted on the patient. The imaging operation thus becomes complex.

Still image capturing is conventionally performed in a combination of frontal imaging and lateral imaging. In a moving-state imaging of a chest, the accuracy of medical diagnosis (image diagnosis) is expected to be heightened if the respiratory moving-state imaging is performed from the front and the side of the patient.

In still image capturing, frontal imaging and lateral imaging are separately performed. The frontal image and the lateral image are then juxtaposed to each other for examination in diagnosis. Since imaging is performed in an inspiration mode in still image capturing, the frontal image and the lateral image phase match each other in the respiration cycle. However, in motion image capturing, if motion images are displayed in the order of capture with the frontal image and the lateral image juxtaposed, the frontal image and the lateral image fail to match each other in phase for the reason mentioned above. This presents difficulty in associating the frontal image and the lateral image in diagnosis.
The present invention is provided as a solution.
The method of processing image data and displaying a motion image considered herein is a method of the kind such as that disclosed by European Patent Application EP-A-1,061,474 in which processing is performed for a plurality of images which have been captured successively and which each exhibit a geometric feature that is cyclically variable, the method comprising steps of: extracting from image data the geometric feature value for each image amongst the plurality of images; and displaying the motion image.
The method disclosed by this reference is applied to images of a heart it is useful as a means are studying movements of the heart and the phase of movement of different portions of the heart muscle.
The method of the present invention is applied to either (a) images of a lung and the geometric feature that is extracted is a lung field parameter being either a lung field area or a lung field height; or (b) images of a limb joint and the geometric feature that is extracted is an angle of bend of the limb joint.
Characteristically, the method of the present invention includes steps of: classifying each of the images amongst a plurality of groups in dependence upon the relative feature values extracted for successive ones amongst the images; sorting the images in each group in order of geometric feature value; and displaying the sorted images of each group, in turn, so as to display the motion image.
In another aspect the present invention provides an apparatus for processing image data and displaying a motion image, for a plurality of images which have been captured successively and which each exhibit one of the following geometric features: (a) a lung field parameter being either a lung field area or a lung field height; or, (b) an angle of bend of a limb joint; the apparatus comprising: extraction means for extracting the geometric feature value for each image amongst the plurality of images; classifying means for classifying each of the images amongst a plurality of groups in dependence upon the relative feature values extracted for successive ones amongst the images; sorting means for sorting the images in each group in order of feature value; for display by the display means in respective groups in turn so as to display the motion image; and display means adapted to display the sorted images of each group in turn, to display a motion image.
The present invention provides a program product including instructions for causing a computer to process image data and control display of a motion image, for a plurality of images which have been captured successively and which each exhibit one of the following geometric features: (a) a lung field parameter being either a lung field area or a lung field height; or, (b) an angle of bend of a limb joint, which instructions are executable to cause the computer to perform the following steps of: extracting from image data a feature value for each image amongst the plurality of images; classifying each of the images amongst a plurality of groups in dependence upon the relative feature values extracted for successive ones amongst the images; sorting the images in each group in order of feature value; and controlling display means to display the sorted images of each group, in turn, so as to display a motion image.
The aforesaid program product may consist in a computer-readable storage medium storing program instructions.
Features and advantages of the present invention, and preferred embodiments thereof, will be apparent from the following description taken in conjunction with the accompanying drawings, in which like reference characters designate the same or similar parts throughout the figures thereof.
The accompanying drawings, which are incorporated in and constitute a part of the specification, illustrate embodiments of the invention and, together with the description, serve to explain the principle of the invention.
FIG. 1 is a block diagram illustrating a system of the present invention;
FIG. 2 illustrates an example of imaging results;
FIG. 3 is a flow diagram illustrating an image processing by an image processor;
FIG. 4 is a histogram of a frontal chest image;
FIG. 5 is a graph illustrating the relationship between a cumulative histogram and a linear regression line thereof;
FIG. 6 illustrate an error between the cumulative histogram and the linear regression line thereof;
FIG. 7 is a histogram of a frontal chest image below a threshold value of 1;
FIG. 8 is a diagrammatic view of the frontal chest image;
FIG. 9 is a flow diagram illustrating a frontal image phase matching and display process;
FIG. 10 is an display example of frontal and lateral motion images;
FIG. 11 diagrammatically illustrates a moving-state image of a knee joint which performs a bending and unbending practice;
FIG. 12 diagrammatically illustrates a feature value calculation algorithm for a knee joint moving-state imaging;
FIG. 13 is a graph illustrating a chronological change in the feature value;
FIG. 14 illustrates a display example of a knee joint moving-state imaging;
FIG. 15 is a block diagram illustrating the construction of a computer that executes a program of a function or an operation of one embodiment of the present invention;
FIG. 16 illustrates one embodiment in which the present invention is implemented in a system linked to a network;
FIG. 17 is a flow diagram illustrating the flow of the process of an imaging system; and
FIG. 18 is a flow diagram illustrating the flow of the process of a diagnosis request management system.

### DETAILED DESCRIPTION OF THE PREFERRED EMBODIMENTS

Preferred embodiments of the present invention will be described in detail in accordance with the accompanying drawings.

### First Embodiment

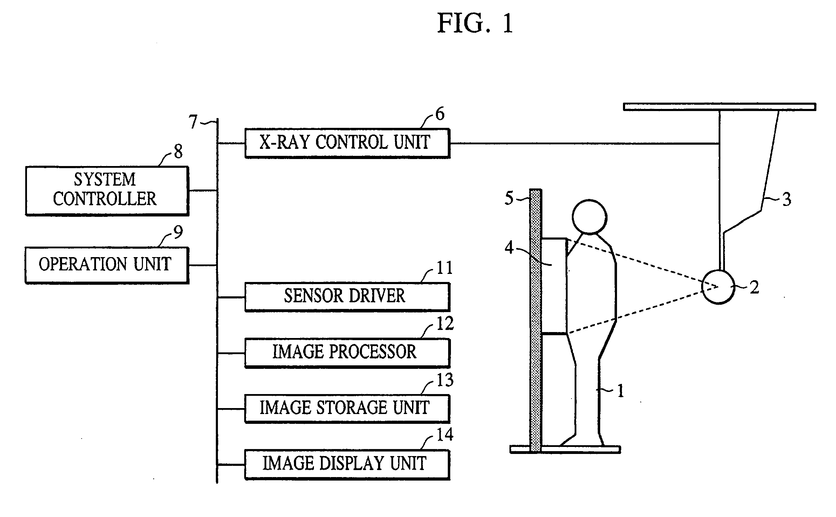

FIG. 1 illustrates a system of one embodiment of the present invention. Referring to FIG. 1, there is shown a two-dimensional sensor 4 formed of an amorphous semiconductor and a phosphor screen. The pixel size of the sensor 4 is 160 µm x 160 µm, and the number of pixels thereof is 2688 x 2688. The sensor 4, here used for imaging with a patient upright, is supported by a stand 5.

An X-ray tube 2 is supported by a ceiling suspension 3, and is movable to match the body size of a patient 1 (also referred to as a subject or an object). The X rays emitted from the X-ray tube 2 are transmitted through the patient and reaches the sensor 4. The X rays are converted by the phosphor screen into visible light which is then converted into image data through the amorphous semiconductor. An X-ray operator then instructs an operation unit 9 to issue the command to start imaging. In response to the command, a system controller 8 controls an X-ray control unit 6 and a sensor driver 11, resulting in an X-ray image.

The respiration cycle of the patient 1 contains an inspiratory mode and an expiratory mode. The inspiratory mode refers to the mode in which the patent 1 inspires, and during the inspiratory mode, the area of the lung field expands in the chest and the diaphragm is pushed down. During the expiratory mode, the patient 1 expires, the area of the lung field contracts, and the diaphragm is pushed up.

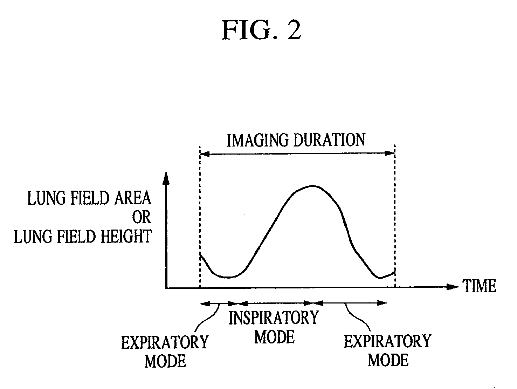

The respiration cycle refers to one cycle of the respiration composed of one expiratory mode and one inspiratory mode. As already discussed, it is technically difficult to exactly complete one respiration cycle within the duration of a series of X-ray exposures in response to a command of the X-ray operator from the operation unit 9. FIG. 2 shows one example of imaging. Referring to FIG. 2, the abscissa represents time, and the ordinate represents the area of the lung field or the distance between the apex of the lung to the diaphragm (the height of the lung). An imaging duration during which the X rays are directed in response to the command of the operator from the operation unit 9 includes an end portion of an expiratory mode, an entire inspiratory mode, an entire expiratory mode, and the initial portion of an inspiratory mode.

Even if the operator instructs the patient 1 to control the respiration cycle, it is difficult for the patient 1 to respire as instructed. Controlling the respiration cycle is not as easy as merely holding the breath.

In this embodiment, three X-ray pulses a second are emitted, and images responsive to the X-ray pulses are captured. When the imaging is performed with the respiration cycle for 10 seconds, 5 seconds for the inspiration mode and 5 seconds for the expiration mode, the resulting number of images is 30.

The captured images are transmitted to an image processor 12 through the sensor driver 11. The image processor 12 analyzes the images, and arranges the collected images in order, and an image storage unit 13 stores the arranged images. For example, the image processor 12 includes a computer, and the image storage unit 13 is formed of the memory of the computer or a magnetic disk.

An image display unit 14 successively presents stored motion images in response to a command from an operation unit (not shown) operated by an operator. The above-mentioned units are connected to the system controller 8 through a system bus 7. The system controller 8 controls the timing of the driving of the above-mentioned units and the flow of data. The system controller 8 is a computer which operates under the control of a computer program.

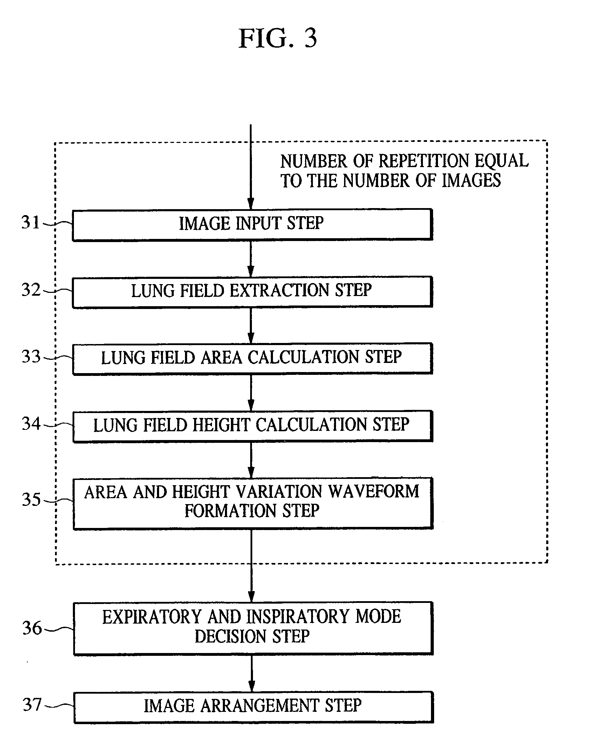

The image processing steps carried out by the image processor 12 will be discussed with reference to the flow diagram illustrated in FIG. 3. If it is assumed that about 30 images (hereinafter after referred to as N images) are obtained during the respiration cycle illustrated in FIG. 2, N images are fed to the image processor 12 (step 31). A lung field extraction step is performed (step 32).

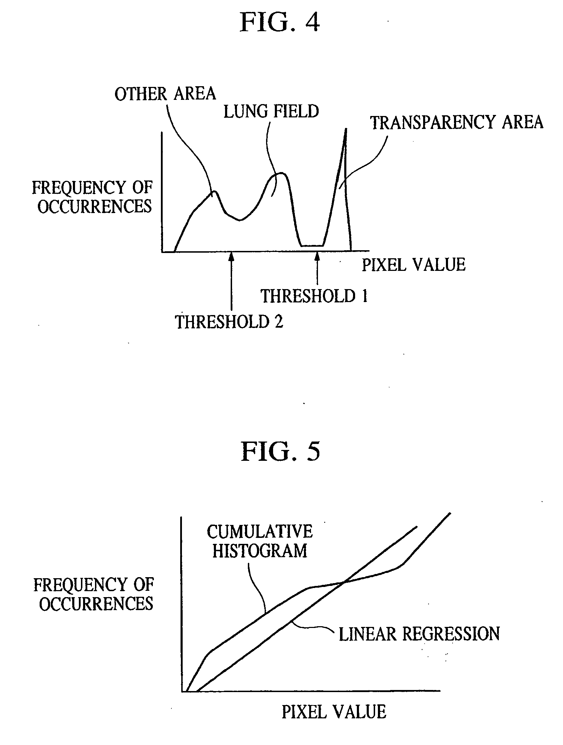

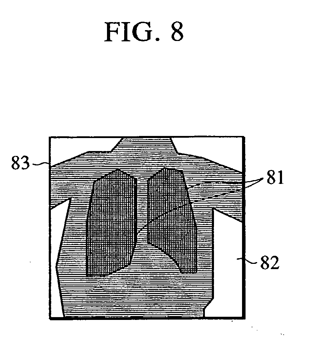

In the lung field extraction step 32, the lung field is extracted from the frontal chest image. FIG. 4 is a histogram of a typical frontal chest image. The histogram includes three peaks. The three peaks correspond to a lung field area 81, an X-ray transparency area 82, and other area 83 (the mediastinal part, the heart, the subdiaphragmatic area, etc) as shown in Fig. 8. To identify the lung field, the image is binarized by determining whether a pixel value is between a threshold 1 and a threshold 2.

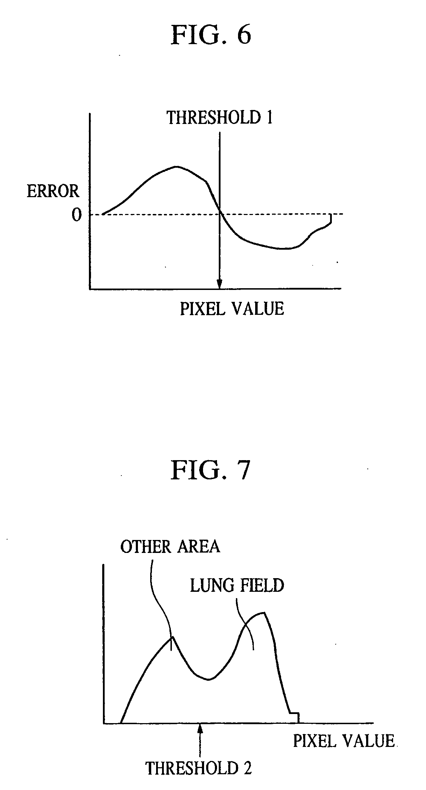

FIG. 5 shows a cumulative histogram corresponding to a histogram in FIG. 4, and the linear regression line of the cumulative histogram. Empirically, the pixel value at the intersection of the cumulative histogram and the linear regression line is the threshold 1. Specifically, referring to FIG. 6, the error (the difference) between the cumulative histogram and the linear regression line is calculated, and the zero crossing point of the error indicates the threshold

### 1. The threshold 1 is thus determined.

The area having the pixel value equal to or larger than the threshold 1 is removed from the image. The histogram of the remaining area is calculated. The histogram shown in FIG. 7 is the result of the calculation. The difference between the cumulative histogram (not shown) of the histogram in FIG. 7 and the linear regression line thereof (not shown) is calculated, and the zero crossing point of the difference is thus determined. The pixel value at that point generally corresponds to the threshold 2. The image is binarized so that the pixel value falling between the threshold 1 and the threshold 2 is 1 with the other pixel value being zero. The lung field is thus extracted. The binarized image is referred to as a binarized lung field image.

The area S of the lung field is calculated by counting the number of pixels with a pixel value 1 of the binarized lung field image (step 33). In succession, the projection of the binarized lung field image in the vertical direction is calculated. Based on the projection, the range of each of the left lung and the right lung is calculated. The projection of each of the left lung image and the right lung image in the horizontal direction is calculated. In this way, the vertical lengths of the projections are obtained as the height of the right lung HR and the height of the left lung HL (step 34).

The area of the lung field S, the right lung height HR, and the left lung height HL calculated for N input images are plotted in a graph. The waveform illustrated in FIG. 2 results (step 35). Besides connecting calculated points with lines in plotting, these points may be interpolated using the spline function. A duration within which the lung field area S increases is defined as an inspiratory mode time and a duration within which the lung field area S decreases is defined as an expiratory mode time. A determination is made of which mode time each image belongs to (step S36). The image processor 12 associates each image with the determined mode, and the calculated lung field area, or the lung field height as phase information of the image and stores the phase information in the memory thereof or in the image storage unit 13. The phase information is stored as a portion of header information of the motion image data. The phase refers to information representing a stage in which the process of a moving state lies in the series of moving states of at least a partial region of the object.

The images are arranged using the determined mode, and the calculated lung field area, or the lung field height (the phase information) (step 37). Specifically, the images are divided into the inspiratory mode and the expiratory mode, and then arranged in the order of from small to large lung field area in the inspiratory mode, or in the order of from large to small lung field area in the expiratory mode. Finally, the N images are arranged from the inspiratory mode to the expiratory mode. The arranged images are displayed on the image display unit 14 as a motion image automatically, or manually in response to the operation of the operator (or an engineer or a physician).

In the above discussion, the arrangement of the images is performed depending on the lung field area. Alternatively, the images are arranged in accordance with the lung height of one lung or the two lungs, or one of the two lungs whichever is higher in height. In the above embodiment, the N images are arranged in the order of from the inspiratory mode to the expiratory mode. Alternatively, the N images may be arranged in the order of from the expiratory mode to the inspiratory mode.

In the above arrangement, image data may be collected at any phase of the respiration cycle. The images are arranged based on the phase of the respiration cycle. This arrangement provides a respiratory motion image (a respiratory moving-state image) which is not affected much by a difference of the phase of the respiratory cycle depending on the timing of the imaging start. The physician can easily diagnose the patient using such a respiratory motion image, thereby shortening diagnosis time.

### Second Embodiment

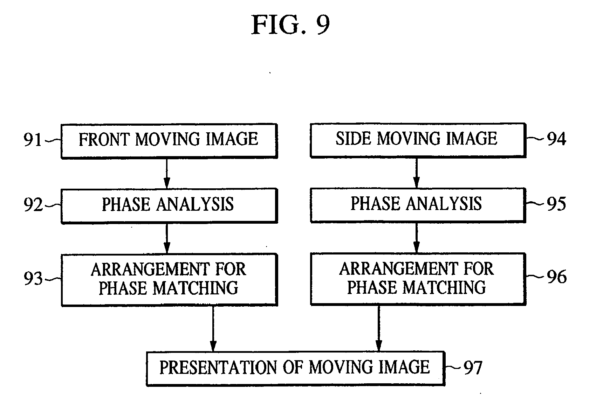

In a second embodiment, the arrangement of the first embodiment is applied to the combination of frontal and lateral chest images. Referring to the flow diagram illustrated in FIG. 9, the arrangement of the first embodiment is used. A frontal chest image is input (step 91), phase analysis is performed based on an extracted lung field (step 92), and the arrangement of the images is performed for phase matching (step 93). The lateral chest image is similarly processed. A lateral chest image is input (step 94), phase analysis is performed based on the extracted lung field (step 95), and an arrangement of the images is performed for phase matching (step 96). If the frontal chest images are arranged in the order of from the expiratory mode to the inspiratory mode, the lateral chest images are also arranged in the order of from the expiratory mode to the inspiratory mode. When the arrangement of the frontal motion image and the lateral motion is complete, the motion image is displayed (or is ready to be displayed) (step 97).

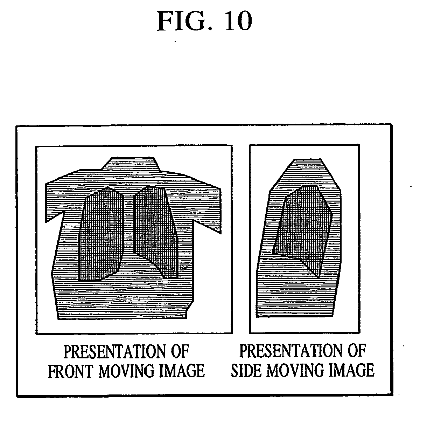

FIG. 10 illustrates an example of a motion image screen presented on a display unit. The display unit provides a motion image display window for the frontal chest image on the left-hand side thereof, and a motion image display window for the lateral chest image on the right-hand side thereof. When the start command of the motion image display is issued through a user interface (not shown), the display unit switches between a frame forming the frontal motion image and a frame forming the lateral motion image with phase matched to present the frontal and lateral images.

It should be noted that the numbers of the frontal chest images and the lateral chest images are occasionally different from each other from the expiratory mode to the inspiratory mode. As already discussed, the frontal chest imaging and the lateral chest imaging are carried out at different times. Depending on the expiration of the patient during imaging, the imaging time duration of the expiratory mode may be different from the imaging time duration of the inspiratory mode. For example, the number of images may be as follows: 17 frontal chest images in the inspiratory mode, 13 frontal chest images in the expiratory mode, 15 lateral chest images in the inspiratory mode, and 15 lateral chest images in the expiratory mode. The imaging time is 10 seconds for each of the frontal imaging and the lateral imaging. The display time is set to any length. In medical diagnosis, a physician may select between a 5 second display, for example, for cursory examination and a 20 second display, for example, for detailed examination. When the 5 second display is selected, the frontal image and the lateral image are respectively presented for 5 seconds. The frontal image is in the inspiratory mode while the lateral image is transitioned into the expiratory mode. Under such circumstances, the purpose of the juxtaposition of the frontal image and the lateral image is not fully achieved so that the physician cannot diagnose the patient efficiently.

When the display time is set to 5 seconds, the inspiratory mode is set to 2.5 seconds, and the expiratory mode is also set to 2.5 seconds, and thus the non-coincidence does not take place in the phase relationship between the frontal image and the lateral image. Specifically, when 17 inspiratory mode frontal images are displayed within 2.5 seconds, the frame rate of the motion image in the inspiratory mode is 17/2.5 fps. When 13 expiratory mode frontal images are displayed within 2.5 seconds, the frame rate of the motion image in the expiratory mode is 13/2.5 fps. The frame rate of the lateral images is 15/2.5 fps for each of the expiratory mode and the inspiratory mode.

In the above example, the display time of 5 seconds is evenly divided between the expiratory mode and the inspiratory mode. It is not necessary to set the same time for the expiratory mode and the inspiratory mode. The ratio of time division may be changed. For example, more time is allowed for the inspiratory mode for a detailed examination, and less time is set for the expiratory mode for a cursory check if such a setting is diagnostically meaningful. Depending on the type of the disease, the time division ratio may be preset, and the division ratio may be selected based on disease information input from a diagnosis report input unit (not shown).

The chest respiratory image has been previously discussed. The present invention is not limited to chest respiratory imaging. The present invention is also applicable to the left and right breast X-ray imaging. Conventionally, the breast X-ray imaging is performed for examination using a still image. The advancement of semiconductor sensors allows a motion image to be captured. For example, the motion image of the breasts is captured when a pressure plate is moved against each of the breasts so that each breast rolls. Such a motion image visualizes a three-dimensional structure of a calcification or a tumor, if any, and helps physicians to determine whether a lesion is malignant or benign.

Displaying the images of the left and right breasts in synchronization has the following significance. The structure and several parts of the human body are bilaterally symmetrical. For example, the left and right lungs, the left and right eyegrounds, and the distributions of the mammary glands and fat of the left and right breasts are bilaterally symmetrical. During diagnosis, the physician conventionally checks for an unbalance (such as asymmetry) of the left and right images (such as the left and right breast images) to detect or recognize an irregularity. In the above example, when breast imaging is performed with the pressure plate moving and thus with the breast rolling, the X ray incident directions to the breasts are set substantially the same (namely, bilaterally symmetrical) so that the tissues of the breasts similarly appear in image. The difference (or a lesion) between the left and right breasts may be detected or easily recognized.

In the above example, a plurality of motion images of the same object or a pair of objects are captured around the same time, and is then subjected to phase matching for display. The present invention is not limited to images that are captured around the same time. For example, in a screening observation using a still image, comparison with past images is typically performed. When the screening observation or progress monitoring is performed using the motion images, the motion images captured in the past and the motion images currently captured are preferably compared with each other with phases thereof matched. As already discussed, the images are arranged in accordance with the phase, and the phase matched images are presented. When the number of images (frames) constituting the past motion image and the current motion image, the imaging times, and the imaging time intervals between images are different from each other, not only the arrangement of the images but also the adjustment of the switching timing of the images for presenting the motion image (such as the frame rate) is required. It is important that the display schedule (display timing) on a time scale be determined in accordance with the phase information of each image constituting the motion image. The above arrangement (sorting) of the images and the adjustment of the frame rate are one of the manners of the determination of the display schedule on the time scale. Instead of physically arranging the images stored in the memory, the schedule information such as the display sequential order or the display timing of the images is stored together with motion image data with the schedule information associated with each image. The motion image is then presented in accordance with the display schedule information.

In the above example, the two motion images are phase matched. The present invention is not limited to the two motion images. Three or more motion images including a past motion image may be phase matched for display. In this case, a single motion image is selected from a plurality of motion images, and the remaining motion images are phase matched with the selected motion image serving as a reference. For example, when a current motion image is used for diagnosis, the current motion image preferably serves as a reference for phase matching.

The physician thus can easily diagnose a disease when the frontal chest image and the lateral chest image are presented as a motion image in juxtaposition in accordance with the above embodiment, because the phases of the images match each other. With the display time of the expiratory mode and the display time of the inspiratory mode in the chest adjustable, a display appropriate for examining the disease is presented. Besides the chest image, the present invention is applicable to the left and right moving-state breast X-ray imaging. Diagnosis is facilitated taking advantage of bilateral symmetry of mammary glands or fat distributions of the left and right breasts.

### Third Embodiment

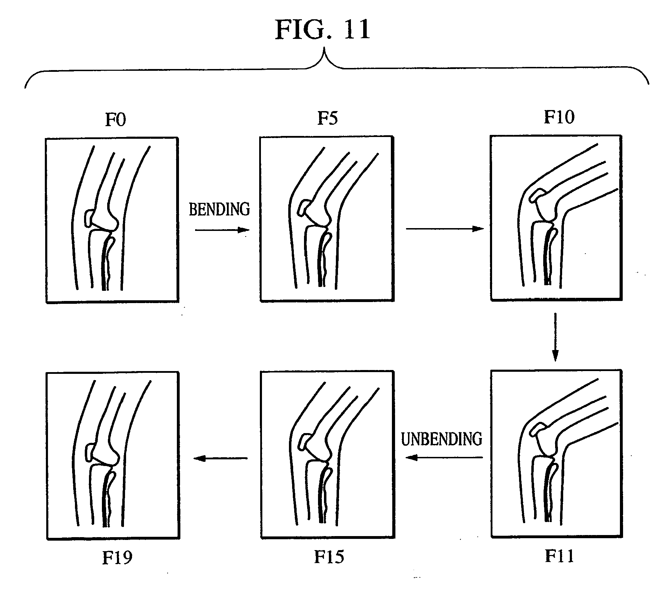

Moving-state imaging is effective in the diagnosis of the abdomen performing the abdominal breathing, the waist or one of the extremities including the joints performing a bending and stretching exercise, or an area of the human body performing an exercise. FIG. 11 illustrates moving-state images of a knee joint performing a bending and stretching exercise. As shown, the knee joint is stretched at state F0, gradually bends as in F5, F10 and F11, then gradually stretches as in F15 and reaches a fully stretched state F19. In this moving-state imaging, a total of 20 frames is obtained. Phase analysis in the knee joint bending and stretching exercise is discussed below. In the analysis of the moving-state chest, the lung field area is used as a feature value. In the phase analysis of the knee joint performing a bending and stretching exercise, a different feature value is introduced. In the phase analysis, an appropriate feature value is used depending on the area of an object for the moving-state imaging.

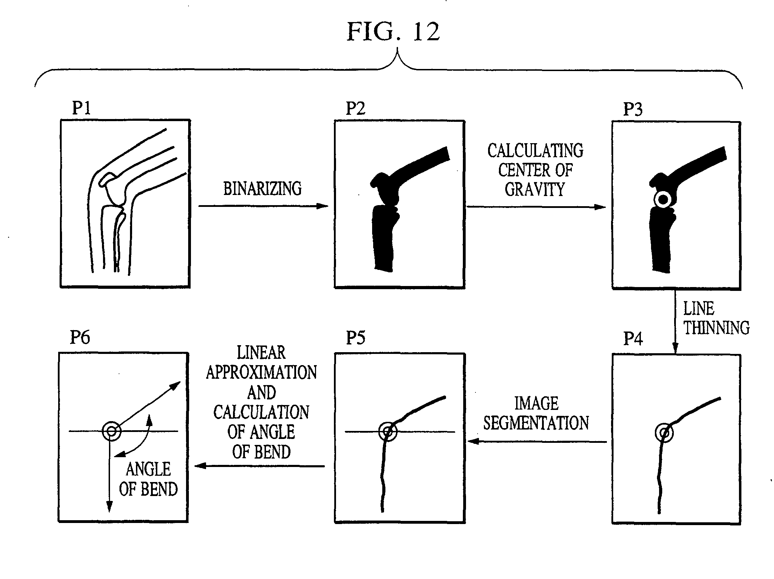

An angle of bend made between the upper leg (thigh) and the lower part of the leg is used as a feature value in the bending and stretching exercise of the knee joint. An example of algorithm (in a flow diagram) for calculating the angle is discussed with reference to FIG. 12. First, a knee joint moving-state image is input to the image processor 12 (step P1). Each frame of the moving-state image is binarized, and a binarized image representing the presence of a bone structure is obtained (P2). The threshold value in the binarizing operation is determined from a zero crossing point of the difference between a cumulative histogram of image data and an approximate line thereof as in the preceding method (see FIG. 5). The determination of the threshold does not need a high accuracy, but an adjustment may be required depending on the ratio of the entire radiation exposure area to the object area.

The center of gravity of the binarized image obtained in step P2 is calculated (step P3). It is known from experience that the center of gravity is present in the area of the knee joint. The position of the center of gravity is represented by a ring as shown. The binarized image is then represented in a thin line by a morphological operation (step P4). The thin line representation is shown in P4. The thin-line image is then segmented at the center of gravity into two, one upper image and the other lower image (step P5). The segmented image is shown in P5. Finally, the bone portions represented in the thin line in the upper image and the lower image are approximated in straight lines, and the angle (of bend) made by the two straight lines is calculated (step P6). The approximation by the straight lines is performed in the following way.

1) The projections of the thin line in the X direction and the Y direction are calculated for each of the upper image and the lower image.

2) The ends of each projection are determined.

3) The ends of the thin line are determined from the ends of the projection for each of the upper image and the lower image, and a line passing both ends of each thin line is treated as a line approximating each bone.

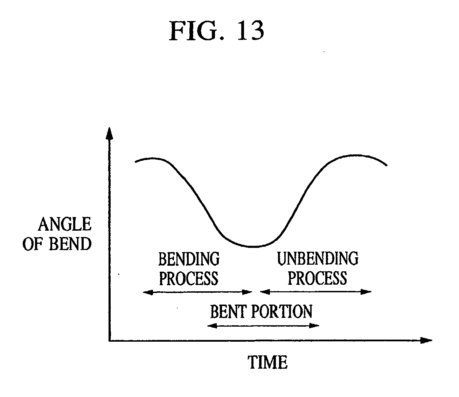

The angle of bend made between the two lines simulating the two bones (the upper and lower leg bones) is calculated in this way. When the angle of the bend is calculated in the input image as shown in FIG. 11, a graph plotting the angles of the bend is obtained as shown in FIG. 13. The exercise is sorted into the bending process in which the angle of bend decreases and the unbending process in which the angle of bend increases. An area where the angle of bend is relatively small is called a bent portion (a threshold of the angle of bend is set based on experience). Using such analysis (classifying) results, particular areas only, such as the bending process (bending area), and the unbending process (unbending area), and the bent portion (bent area) can be selectively displayed, and thus serve diagnosis purposes.

Using the above-referenced analysis results, a display rate in a particular phase region of the moving-state image is made different. Rather than displaying the entire moving-state image at a time scale which has been used at the imaging time, the phase region in the bent portion is replayed at a time scale half the rate of the imaging, and the phase region is easily observed without prolonging time required image displaying.

In another example of display rate control, the display rate may be controlled in every sorted phase region. Alternatively, the display rate may be controlled depending on a rate of change in the angle of bend. Specifically, the display rate may be updated in accordance with the rate of change in the angle of bend shown in FIG. 13. For example, if the display rate is changed in inverse proportions to the rate of change in the angle of bend, the physician can observe the moving-state image with the rate of change in the angle of bend remaining constant. It will be perfectly acceptable that the switching rate of image is set to be slow when the rate of change in the angle of bend is large, and that the switching rate of image is set to be fast when the rate of change in the angle of bend is small. Specifically, let Δ represent the rate of change in the angle of bend between frames of the moving-state image, the display rate is controlled so that the display interval of the images is K·Δ ms (K is a constant).

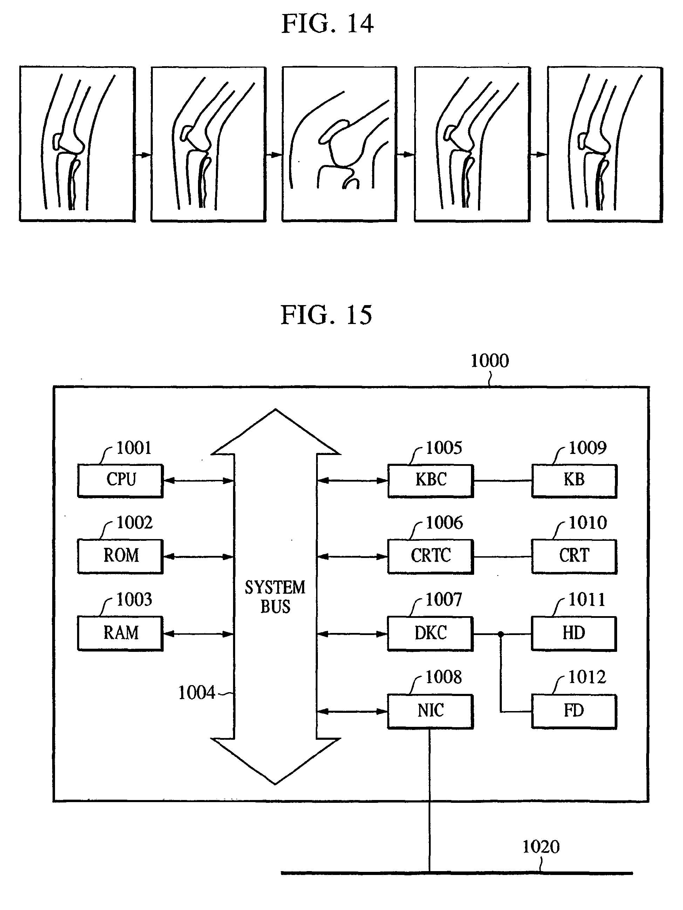

Using the above-referenced analysis results, a particular phase region of the moving-state image only (for example, the process of interest) is expanded to be displayed. For example, referring to FIG. 14, the knee in the bent portion is expanded. The physician thus observes the moving-state image in the phase region of interest in detail while observing the entire moving-state image. The area to be enlarged may be automatically set in accordance with the image data, or may be manually set by the operator through a user interface (not shown). For example, in the knee joint moving-state image, the knee is automatically set as an area to be enlarged, using the calculation result of the center of gravity.

### Alternate Embodiments

A storage medium storing a program code of software program performing the function of each of the first through third embodiments may be supplied in an apparatus or a system. A computer (a CPU or an MPU) in that apparatus or that system reads the program code from the storage medium and performs the function. The object of the present invention is thus achieved.

The program code itself read from the storage medium performs the function of each of the first through third embodiments. The storage medium storing the program code and the program code itself fall within the scope of the present invention.

Available as storage media for feeding the program code are ROM (Read-Only Memory), a floppy disk (Trademark), a hard disk, an optical disk, a magneto-optical disk, a CD-ROM (Compact Disk- ROM), a CD-R (Recordable CD), a magnetic tape, a nonvolatile memory card, and the like.

By executing the program code read by the computer, the function of each of the first through third embodiments is performed. Furthermore, the process in whole or in part of the above embodiments is performed in cooperation with the OS (operating system) running on the computer according to the instruction of the program code. Through the process, the function of one of the first through third embodiments is carried out. Such a program code falls within the scope of the present invention.

The program code from the storage medium is read into a memory incorporated in a feature expansion board in the computer or in a feature expansion unit connected to the computer. The CPU mounted on the feature expansion board or the feature expansion unit performs partly or entirely the actual process in response to the instruction from the program code. The function of each of the first through third embodiments is executed through the process. Such a program code falls within the scope of the present invention.

When the present invention is applied to the program or the storage medium storing the program, such a program is formed of the program codes corresponding to one of the flow diagrams illustrated in FIGS. 3, 9, and 12.

FIG. 15 illustrates the construction of such a computer 1000.

Referring to FIG. 15, the computer 1000 includes a CPU 1001, an ROM 1002, an RAM 1003, a keyboard controller (KBC) 1005 for controlling a keyboard (KB) 1009, a CRT controller (CRTC) 1006 for controlling a CRT display (CRT) 1010, a disk controller (DKC) 1007 for controlling a hard disk (HD) 1011 and a floppy (Trademark) disk (FD) 1012, and a network interface controller (NIC) 1008 for connection with a network 1020, with all these blocks mutually interconnected through a system bus 1004 for communication.

The CPU 1001 generally controls the blocks connected to the system bus 1004 by reading and executing a software program stored in one of the ROM 1002 and the hard disk (HD) 1011, or a software program stored in the FD 1012.

The CPU 1001 performs the function of each of the first through third embodiments by reading a process program in accordance with a predetermined process sequence from one of the ROM 1002, the HD 1011, and the FD 1012.

The RAM 1003 serves as a main memory or working memory for the CPU 1001. The KBC 1005 controls the KB 1009 or other pointing device (not shown) for command input. The CRTC 1006 controls the CRT 1010 for displaying.

The DKC 1007 controls access to the HD 1011 and the FD 1012, each of which stores a boot program, a variety of application programs, an edit file, a user file, a network management program, and a predetermined process program.

The NIC 1008 bilaterally exchanges data with an apparatus or a system over the network 1020.

The present invention is applicable to a system including a plurality of apparatuses (such as a radiation generator, a radiation imaging apparatus, an image processor, interfaces, etc.) or a standalone apparatus in which the functions of these apparatuses are integrated. When the present invention is applied to the system composed of a plurality of apparatuses, the plurality of apparatuses are connected to each other through electrical (communication) means, optical (communication) means and/or mechanical interconnect means.

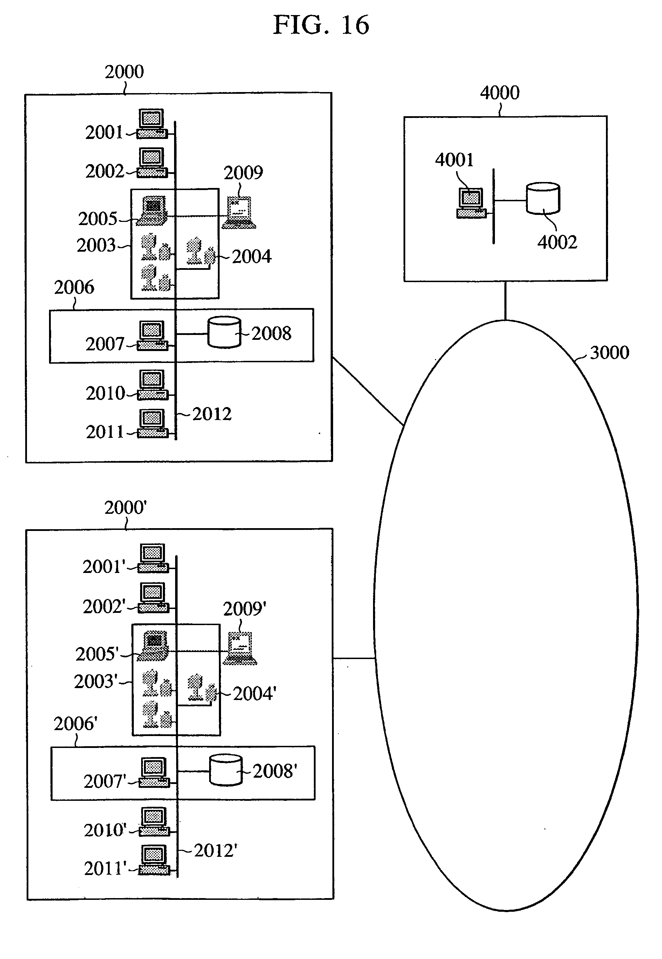

The present invention is applicable to an image diagnosis assisting system connected to a network (such as LAN and/or WAN). Referring to FIG. 16, there are shown a medical institution 2000, and a hospital information system (hereinafter referred to as HIS) 2001 including a computer or a computer network that manages information of a patient who has received a medical service (such as medical record, examination results, billing information, etc.). A department of radiology information system (hereinafter RIS) 2002 includes a computer or a computer network that manages information of a department of radiology. For example, the RIS 2002 manages radiation imaging request information from the HIS in cooperation with an imaging system 2003 to be discussed later.

The imaging system 2003 performs radiation imaging. For example, the imaging system 2003 includes at least one imaging apparatus 2004 that performs radiation imaging on a patient and outputs image data, and an imaging management/image processor server 2005 that manages the radiation imaging based on the imaging request information from the RIS and/or processes radiation images. Each of the imaging system 2003 and the imaging apparatus 2004 includes the system shown in FIG. 1.

A Picture Archiving and Communication System (hereinafter referred to as PACS) 2006 archives image data from the imaging system 2003 together with information (also called supplementary information) required for the management and/or image diagnosis of the image data, and provides the image data (and the supplementary information) as necessary. The PACS 2006 includes, for example, a PACS server 2007 including a computer or computer network, and an image storage apparatus 2008 for storing the image data and the attached information.

In cooperation with the imaging system 2003 and/or the PACS 2006, a diagnosis request management system 2009 sends, to a diagnostician, diagnosis request information of the image data obtained from the imaging system 2003 to furnish the diagnostician with the image data (for image diagnosis), automatically or in response to an operator (or radiation engineer), while also managing the progress of the image diagnosis. The diagnosis request management system 2009 includes a computer or a computer network.

Diagnosis terminals 2010 and 2011 (image viewers), used by diagnosticians, includes a computer or a computer network which receives the diagnosis request information from the diagnosis request management system 2009, acquires the image data and the supplementary information from the PACS 2006, receives diagnosis results input by the diagnostician, and sends diagnosis result information and/or diagnosis end information to the diagnosis request management system 2009.

The elements 2001-2011 are interconnected to each other through a LAN (Local Area Network) 2012. The diagnosis result information is sent from the diagnosis request management system 2009 or directly from the diagnosis terminals 2010 and 2011 to at least one of the hospital information system 2001, the radiology information system 2002 and the PACS 2006.

The destination of the diagnosis request from the diagnosis request management system 2009 is not limited to within the medical institution 2000. Through a public telephone line or WAN (Wide Area Network), a diagnosis request may be sent to a diagnostician at another medical institution. FIG. 16 shows an example in which the medical institution 2000 is linked to a medical institution 2000' through the Internet 3000. Like the medical institution 2000, the medical institution 2000' here also includes elements 2001'-2012'. The present invention is not limited to this arrangement. The diagnosis request management system 2009 in the medical institution 2000 sends a diagnosis request to the medical institution 2000' through the Internet 3000 and the diagnosis request management system 2009' in the medical institution 2000', and then obtains the diagnosis results from the medical institution 2000'.

A system using a diagnosis agency 4000 may be established instead of a system which directly exchanges the diagnosis request information, the image data and the diagnosis result information between the medical institutions. In this case, the diagnosis request management system 2009 in the medical institution 2000 sends the diagnosis request information containing the image data to the diagnosis agency 4000. The diagnosis agency 4000 is owned by a diagnosis service institution (or a diagnosis service company), and includes an agency server 4001 including a computer or a computer network, and a storage device 4002 for storing required data.

The diagnosis agency 4001 has the function of selecting a medical institution and/or a diagnostician appropriate for diagnosis based on the diagnosis request information from the medical institution 2000, the function of sending the diagnosis request information to the medical institution and/or the diagnostician, the function of furnishing the medical institution and/or the diagnostician with the image data and the like required for diagnosis, the function of acquiring the diagnosis results from the medical institution and/or the diagnostician, and the function of providing the medical institution 2000 with the diagnosis result information and other information. A storage device 4002 stores the diagnosis request information, and data required for these functions, for example, data required to select a medical institution and/or a diagnostician appropriate for diagnosis (for example, data such as network addresses of medical institutions and/or diagnosticians, fields and level of diagnosis, schedules, etc.). In such a system, the diagnosis request management system 2009 in the medical institution 2000 receives the diagnosis result information from the medical institution and/or the diagnostician appropriate for diagnosis through the Internet 3000 and the diagnosis agency 4000.

The medical institution 2000 is not limited to an institution such as a hospital. For example, the medical institution 2000 may be a health care institution for which a diagnostician works. The medical institution 2000 in this case is replaced with a health care institution 2000" (not shown) composed of the same elements such as the elements 2003-2012. The medical institution 2000 may be a medical examination institution which performs medical examinations only. In this case, the medical institution 2000 is replaced with a medical examination institution 2000"' (not shown) which is composed of the same elements as the elements 2003-2009 and 2012.

A portion of a system, apparatus, means, or function in the medical institution 2000 (for example, the image processor 12 or a portion thereof in the imaging system 2003 or the imaging apparatus 2004) may not be contained in the medical institution 2000, and instead, an identical or similar system, apparatus, means or function in another institution may be used through the Internet 3000.

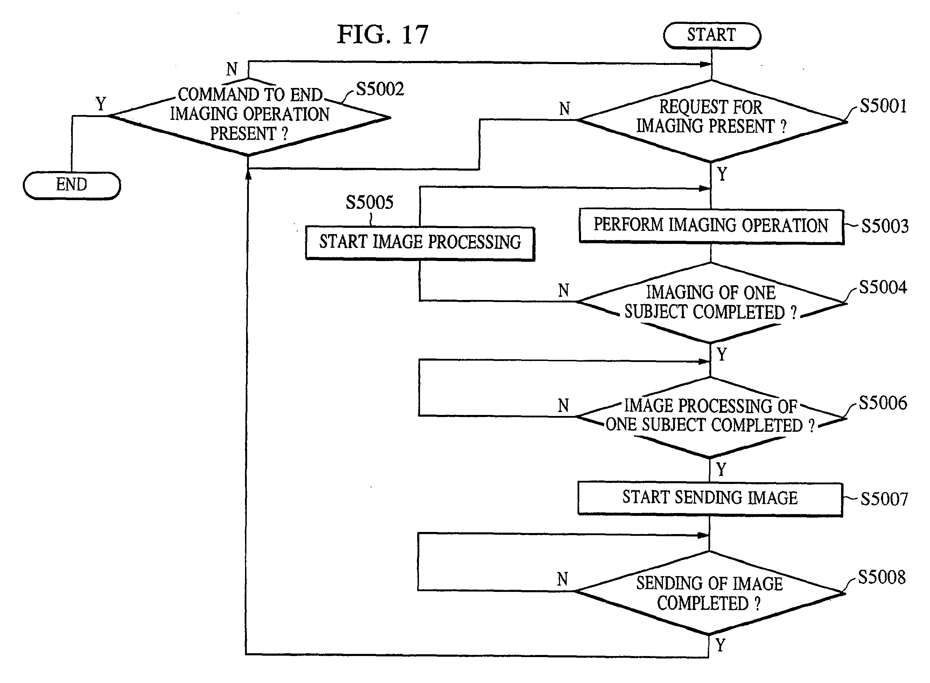

The process flows of the imaging system 2003 and the diagnosis request management system 2009 in the medical institution 2000 are discussed below. The process flow of the imaging system 2003 is discussed first with reference a flow diagram illustrated in FIG. 17. In step S5001, the imaging system 2003 determines the presence or absence of the imaging request information sent from the HIS or RIS. When there is an imaging request information, the algorithm proceeds to step S5003. When there is no imaging request information, the algorithm proceeds to step S5002. The imaging system 2003 determines in step S5002 whether there is an operation end command. If it is determined that there is an operation end command, the imaging system 2003 ends the operation. If it is determined that there is no operation end command, the imaging system 2003 loops to step S5001 to start over again. In step S5003, the imaging system 2003 carries out the imaging as already discussed in each of the above embodiments in response to the imaging request information.

The imaging system 2003 determines whether all requested imaging is completed for a patient (object) subsequent to the imaging (step S5004). If it is determined that the imaging is incomplete, the algorithm loops to step S5003 to continue the imaging after starting image processing on radiation images captured at a preceding cycle in step S5005. The image processing has already been discussed in the above-referenced embodiments, and is carried out in parallel with the imaging process in step S5003. If all imaging for the patient is completed, the algorithm proceeds to step S5006.

The imaging system 2003 determines in step S5006 whether the image processing is completed on all images captured for the patient in the imaging. If it is determined that all images are processed, the algorithm proceeds to step S5007, else the algorithm repeats the determination in step S5006.

In step S5007, the imaging system 2003 starts transmission of all image data subsequent to the image processing of the images of the patient. For example, all image data is transmitted to the PACS 2006, and data used to access the image data transmitted to the PACS 2006 is transmitted to the diagnosis request management system 2009.

In step S5008, the imaging system 2003 determines whether the transmission of the above-mentioned image data is completed. If it is determined that the transmission of the image data is completed, the algorithm loops to step S5002, else the algorithm repeats the determination in step S5008.

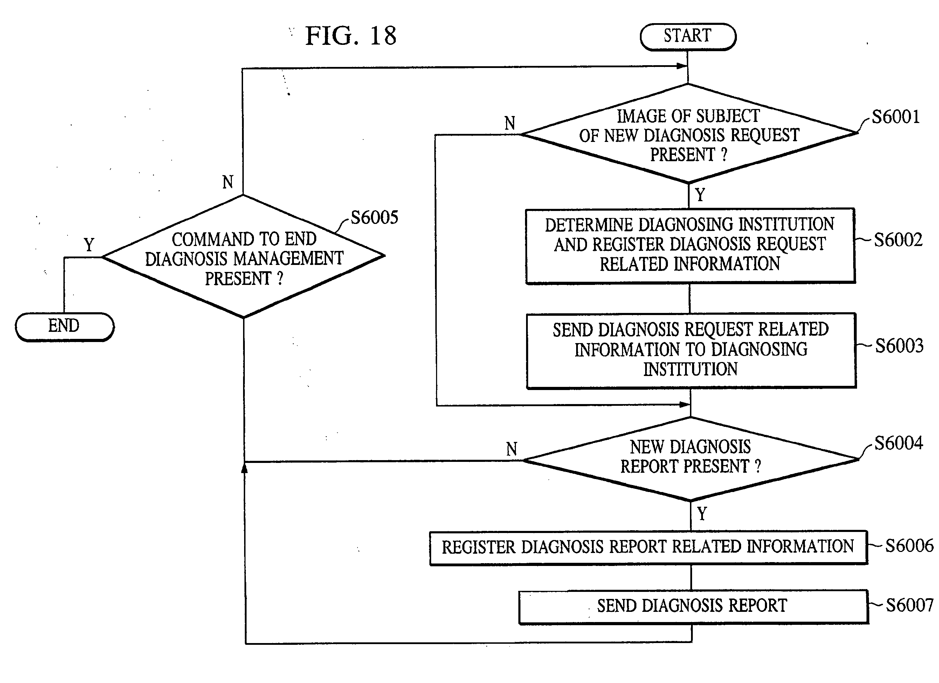

The process flow of the diagnosis request management system 2009 is discussed with reference to a flow diagram illustrated in FIG. 18. In step S6001, the diagnosis request management system 2009 determines the presence or absence of radiation imaging data of a patient for diagnosis. This determination is carried out based on information relating to radiation imaging data of each patient requesting medical diagnosis, transmitted from the imaging system 2003, the other institution 2000', or the diagnosis agency 4000, for example, information for accessing the image data transmitted to the PACS. If it is determined that there is radiation imaging data, the algorithm proceeds to step S6002, else the algorithm proceeds to step S6004.

In step S6002, the diagnosis request management system 2009 determines an institution diagnosing the image which is to be diagnosed, while registering the diagnosis request related information including the diagnosing institution information to manage the progress of the diagnosis. The diagnosing institution is determined based on the information relating to the image to diagnosed, for example, information stored in the storage device relating to the image to be diagnosed as header information of the image data (for example, an area of a patient to be imaged, a method of imaging, the purpose of diagnosis, disease information, designated diagnostician information, etc.). The diagnosing institution may be the other medical institution 2000' or the diagnosis agency 4000. In step S6003, the diagnosis request information containing information for identifying the image to be diagnosed and the image data to be diagnosed is sent to the determined diagnosing institution.

In step S6004, the diagnosis request management system 2009 determines the presence or absence of a new diagnosis report. This determination is performed based on information received from the diagnosis terminal 2010, the other medical institution 2000', or the diagnosis agency 4000. If it is determined that there is a new diagnosis report, the algorithm proceeds to step S6006, else the algorithm proceeds to step S6005. The diagnosis request management system 2009 determines in step S6005 whether there is an operation end command sent thereto. If it is determined that there is an operation end command, the diagnosis request management system 2009 ends the operation, else the algorithm loops to step S6001 to start over again.

In step S6006, the diagnosis request management system 2009 registers the diagnosis report related information (such as the date of acquisition of the diagnosis report, and the content of the report) to manage the progress of the diagnosis. In step S6007, the diagnosis report is transmitted (transferred) to a predetermined destination from among the HIS 2001, the RIS 2002, the PACS 2006, and the computer of the diagnosis requesting institution (including the other medical institution 2000' or the diagnosis agency 4000). The diagnosis request management system 2009 then proceeds to step S6005.

The diagnosis request management system 2009 is formed of a dedicated computer in the above discussion. The present invention is not limited to this arrangement. The function of the diagnosis request management system 2009 may be included in the HIS 2001, the RIS 2002, the imaging management/image processor server 2005 in the imaging system 2003, or the PACS server 2007 in the PACS 2006.
The present invention thus achieves the above-described object as described above.
The present invention is not limited to the above embodiments and various changes and modifications can be made within the scope of the present invention. Therefore to apprise the public of the scope of the present invention, the following claims are made.

## Claims

1. A method of processing image data and displaying a motion image, for a plurality of images which have been captured successively and which each exhibit a geometric feature that is cyclically variable, said method comprising steps of:
extracting (32-34) from image data a geometric feature value for each image amongst said plurality of images; and
displaying the motion image;
**characterised in that**:
(a) said method is applied to images of a lung and the geometric feature that is extracted is a lung field parameter being either a lung field area or a lung field height; or
(b) said method is applied to images of a limb joint performing a bending and stretching exercise and the geometric feature that is extracted is an angle of bend of the limb joint; and
said method also includes steps of:
classifying (35) each of said images amongst a plurality of groups in dependence upon the relative feature values extracted for successive ones amongst said images;
sorting (36) said images in each group in order of feature value; and
displaying (37) the sorted images of each group, in turn, so as to display the motion image.

2. A method according to claim 1 wherein in said step of classifying each of said images, the images are classified into two groups, one group wherein the feature value is increasing, and the other group wherein the feature value is decreasing, in each case for successive images.

3. A method according to claim 1 wherein in said step of classifying each of said images, the images are classified into three groups, one group wherein the feature value is increasing, another group wherein the feature value is decreasing, and yet another group wherein the feature value is of substantially stationary value, in each case for successive images.

4. A method according to any preceding claim, wherein in said step of displaying the sorted images, the display magnification is determined according to sorting by group.

5. A method according to any preceding claim, wherein in said step of displaying the sorted images, the display rate is determined according to sorting by group.

6. An apparatus for processing image data and displaying a motion image, for a plurality of images which have been captured successively and which each exhibit one of the following geometric features: (a) a lung field parameter being either a lung field area or a lung field height; or, (b) an angle of bend of a limb joint;
said apparatus comprising:
extraction means (12) for extracting a geometric feature value for each image amongst said plurality of images;
classifying means (12) for classifying each of said images amongst a plurality of groups in dependence upon the relative feature values extracted for successive ones amongst said images;
sorting means (12) for sorting said images in each group in order of feature value; for display by said display means in respective groups in turn so as to display the motion image; and
display means (12, 14) adapted to display the sorted images of each group in turn, to display a motion image.

7. A program product including instructions for causing a computer to process image data and control display of a motion image, for a plurality of images which have been captured successively and which each exhibit one of the following geometric features: (a) a lung field parameter being either a lung field area or a lung field height; or, (b) an angle of bend of a limb joint, which instructions are executable to cause the computer to perform the following steps of:
extracting (32-34) from image data a geometric feature value for each image amongst said plurality of images;
classifying (35) each of said images amongst a plurality of groups in dependence upon the relative feature values extracted for successive ones amongst said images;
sorting (36) said images in each group in order of feature value; and
controlling display means (12, 14) to display (37) the sorted images of each group, in turn, so as to display a motion image.

8. A computer-readable storage medium storing program instructions, being a program product according to claim 7.

## Patentansprüche

1. Verfahren zum Verarbeiten von Bilddaten und Anzeigen eines bewegten Bildes, für eine Vielzahl von Bildern, welche nacheinander aufgenommen wurden und von welchen jedes ein geometrisches Merkmal besitzt, das zyklisch variabel ist, wobei das Verfahren die Schritte aufweist:
Extrahieren (32 - 34) eines geometrischen Merkmalswerts aus Bilddaten für jedes Bild unter der Vielzahl von Bildern; und
Anzeigen des bewegten Bildes;
**dadurch gekennzeichnet, dass**:
(a) das Verfahren auf Bilder einer Lunge angewendet wird und das geometrische Merkmal, das extrahiert wird, ein Lungenfeldparameter ist, der entweder eine Lungenfeldfläche oder eine Lungenfeldhöhe ist; oder
(b) das Verfahren auf Bilder eines Extremitätengelenks angewendet wird, das eine Beuge- und Streckübung durchführt, und das geometrische Merkmal, das extrahiert wird, ein Beugewinkel des Extremitätengelenks ist; und
das Verfahren auch die Schritte aufweist:
Klassifizieren (35) jedes der Bilder unter einer Vielzahl von Gruppen in Abhängigkeit der relativen Merkmalswerte, die für Nacheinanderfolgende unter den Bildern extrahiert werden;
Sortieren (36) der Bilder in jeder Gruppe in einer Reihenfolge von Merkmalswerten; und
Anzeigen (37) der sortierten Bilder von jeder Gruppe eines nach dem anderen, um das bewegte Bild anzuzeigen.

2. Verfahren gemäß Anspruch 1, wobei in dem Schritt des Klassifizierens jedes der Bilder, die Bilder in zwei Gruppen klassifiziert werden, wobei bei einer Gruppe der Merkmalswert ansteigt und bei der anderen Gruppe der Merkmalswert abnimmt, in jedem Fall für nacheinander folgende Bilder.

3. Verfahren gemäß Anspruch 1, wobei in dem Schritt des Klassifizierens jedes der Bilder, die Bilder in drei Gruppen klassifiziert werden, wobei bei einer Gruppe der Merkmalswert ansteigt, bei einer anderen Gruppe der Merkmalswert abnimmt und bei noch einer anderen Gruppe der Merkmalswert ein im wesentlichen stationärer Wert ist, in jedem Fall für nacheinander folgende Bilder.

4. Verfahren gemäß einem der vorstehenden Ansprüche, wobei in dem Schritt des Anzeigens der sortierten Bilder, die Anzeigevergrößerung gemäß einer Sortierung nach Gruppen bestimmt wird.

5. Verfahren gemäß einem der vorstehenden Ansprüche, wobei in dem Schritt des Anzeigens der sortierten Bilder, die Anzeigerate gemäß einer Sortierung nach Gruppen bestimmt wird.

6. Vorrichtung zum Verarbeiten von Bilddaten und Anzeigen eines bewegten Bildes, für eine Vielzahl von Bildern, welche nacheinander aufgenommen wurden und von welchen jedes eines der folgenden geometrischen Merkmale besitzt: (a) einen Lungenfeldparameter, der entweder eine Lungenfeldfläche oder eine Lungenfeldhöhe ist, oder (b) einen Beugewinkel eines Extremitätengelenks;
wobei die Vorrichtung aufweist:
eine Extrahierungseinrichtung (12) zum Extrahieren eines geometrischen Merkmalswerts für jedes Bild unter der Vielzahl von Bildern;
eine Klassifizierungseinrichtung (12) zum Klassifizieren jedes der Bilder unter einer Vielzahl von Gruppen in Abhängigkeit der relativen Merkmalswerte, die für Nacheinanderfolgende unter den Bildern extrahiert werden;
eine Sortierungseinrichtung (12) zum Sortieren der Bilder in jeder Gruppe in einer Reihenfolge von Merkmalswerten; zum Anzeigen durch die Anzeigeeinrichtung in entsprechenden Gruppen eines nach dem anderen, um das bewegte Bild anzuzeigen; und
eine Anzeigeeinrichtung (12, 14), die angepasst ist, um die sortierten Bilder von jeder Gruppe eines nach dem anderen anzuzeigen, um das bewegte Bild anzuzeigen.

7. Programmprodukt mit Anweisungen, um einen Computer zu veranlassen, Bilddaten zu verarbeiten und ein Anzeigen eines bewegten Bildes zu steuern, für eine Vielzahl von Bildern, welche nacheinander aufgenommen wurden und von welchen jedes eines der folgenden geometrischen Merkmale besitzt: (a) einen Lungenfeldparameter, der entweder eine Lungenfeldfläche oder eine Lungenfeldhöhe ist, oder (b) einen Beugewinkel eines Extremitätengelenks; welche Anweisungen ausführbar sind, um den Computer zu veranlassen, die folgenden Schritte durchzuführen:
Extrahieren (32 - 34) eines geometrischen Merkmalswerts aus Bilddaten für jedes Bild unter der Vielzahl von Bildern;
Klassifizieren (35) jedes der Bilder unter einer Vielzahl von Gruppen in Abhängigkeit der relativen Merkmalswerte, die für Nacheinanderfolgende unter den Bildern extrahiert werden;
Sortieren (36) der Bilder in jeder Gruppe in einer Reihenfolge von Merkmalswerten; und
Steuern einer Anzeigeeinrichtung (12, 14), um die sortierten Bilder von jeder Gruppe eines nach dem anderen anzuzeigen (37), um das bewegte Bild anzuzeigen.

8. Computer-lesbares Speichermedium, das Programmanweisungen speichert, das ein Programmprodukt gemäß Anspruch 7 ist.

## Revendications

1. Procédé de traitement de données d'image et d'affichage d'une image animée, pour une pluralité d'images qui ont été capturées successivement et qui présentent chacune une caractéristique géométrique qui est cycliquement variable, ledit procédé comprenant les étapes qui consistent :
à extraire (32-34) de données d'image une valeur de caractéristique géométrique pour chaque image parmi ladite pluralité d'images ; et
à afficher l'image animée ;
**caractérisé en ce que** :
(a) ledit procédé est appliqué à des images d'un poumon et la caractéristique géométrique qui est extraite d'un paramètre de champ pulmonaire est soit une étendue de champ pulmonaire, soit une hauteur de champ pulmonaire ; ou
(b) ledit procédé est appliqué à des images d'une articulation d'un membre effectuant un exercice de flexion et d'étirement et la caractéristique géométrique qui est extraite est un angle de flexion de l'articulation du membre ; et
ledit procédé comprend aussi les étapes qui consistent :
à classer (35) chacune desdites images dans une pluralité de groupes suivant les valeurs de caractéristiques relatives extraites pour certaines, successives, desdites images ;
à trier (36) lesdites images dans chaque groupe dans un ordre de valeur de caractéristique ; et
à afficher (37) les images triées de chaque groupe, à leur tour, afin d'afficher l'image animée.

2. Procédé selon la revendication 1, dans lequel, dans ladite étape de classement de chacune desdites images, les images sont classées en deux groupes, un groupe dans lequel la valeur de caractéristique est croissante, et l'autre groupe dans lequel la valeur de caractéristique est décroissante, dans chaque cas pour des images successives.

3. Procédé selon la revendication 1, dans lequel, dans ladite étape de classement de chacune desdites images, les images sont classées en trois groupes, un groupe dans lequel la valeur de caractéristique est croissante, un autre groupe dans lequel la valeur de caractéristique est décroissante, et encore un autre groupe dans lequel la valeur de caractéristique est d'une valeur sensiblement stationnaire, dans chaque cas pour des images successives.

4. Procédé selon l'une quelconque des revendications précédentes, dans lequel, dans ladite étape d'affichage des images triées, le grossissement d'affichage est déterminé en fonction du tri par groupe.

5. Procédé selon l'une quelconque des revendications précédentes, dans lequel, dans ladite étape d'affichage des images triées, le taux d'affichage est déterminé en fonction du tri par groupe.

6. Appareil pour le traitement de données d'image et l'affichage d'une image animée, pour une pluralité d'images qui ont été capturées successivement et qui présentent chacune l'une des caractéristiques géométriques suivantes : (a) un paramètre de champ pulmonaire qui est soit une étendue de champ pulmonaire, soit une hauteur de champ pulmonaire ; ou (b) un angle de flexion d'une articulation d'un membre ;
ledit appareil comportant :
un moyen d'extraction (12) destiné à extraire une valeur de caractéristique géométrique pour chaque image parmi ladite pluralité d'images ;
un moyen de classement (12) destiné à classer chacune desdites images parmi une pluralité de groupes en fonction de valeurs de caractéristiques relatives extraites pour certaines, successives, desdites images ;
un moyen de tri (12) destiné à trier lesdites images dans chaque groupe dans l'ordre de valeur de caractéristique ; pour un affichage par ledit moyen d'affichage en groupes respectifs, tour à tour, afin d'afficher l'image animée ; et
un moyen d'affichage (12, 14) conçu pour afficher les images triées de chaque groupe tour à tour, afin d'afficher une image animée.

7. Produit à programme comprenant des instructions pour amener un ordinateur à traiter des données d'image et à commander l'affichage d'une image animée, pour une pluralité d'images qui ont été capturées successivement et qui présentent chacune l'une des caractéristiques géométriques suivantes : (a) un paramètre de champ pulmonaire qui est soit une étendue de champ pulmonaire, soit une hauteur de champ pulmonaire ; ou (b) un angle de flexion d'une articulation d'un membre, lesquelles instructions peuvent être exécutées pour amener l'ordinateur à effectuer les étapes suivantes qui consistent :
à extraire (32-34) de données d'image une valeur de caractéristique géométrique pour chaque image parmi ladite pluralité d'images ;
à classer (35) chacune desdites images dans une pluralité de groupes suivant les valeurs de caractéristiques relatives extraites pour certaines, successives, desdites images ;
à trier (36) lesdites images dans chaque groupe dans un ordre de valeur de caractéristique ; et
à commander un moyen d'affichage (12, 14) pour afficher (37) les images triées de chaque groupe, tour à tour, afin d'afficher une image animée.

8. Support de stockage lisible par ordinateur et stockant des instructions de programme, lequel est un produit à programme selon la revendication 7.
